# EUROPEAN PATENT APPLICATION

(11) **EP 4 546 965 A1**
(43) Date of publication of application: **30.04.2025**
(21) Application number: 23826522.7
(22) Date of filing: 21.06.2023
(51) Int. Cl.: H05K 7/20

(54) **HEAT DISSIPATION SYSTEM AND MEDICAL SYSTEM**

(30) Priority: 21.06.2022 CN 202221564181 U; 24.04.2023 CN 202310450102; 24.04.2023 CN 202320950600 U
(71) Applicant: Shanghai United Imaging Healthcare Co., Ltd., Shanghai 201807 (CN)
(72) Inventor: JIANG, Huaifang, Shanghai 201807 (CN); YAN, Hao, Shanghai 201807 (CN); CHEN, Xing, Shanghai 201807 (CN)
(74) Representative: Murgitroyd & Company
(86) International application number: PCT/CN2023/101750
(87) International publication number: WO 2023/246877

(57) **Abstract**

A heat dissipation system and a medical system are provided. The heat dissipation system includes a circulation pipeline and a cooling device. The circulation pipeline is configured for conveying a cooling medium to a medical system. The medical system includes at least two electric units, and the at least two electric units have different rated cooling temperatures. The cooling device is in communication with the circulation pipeline and is configured for adjusting a temperature of the cooling medium, so as to dissipate heat of the at least two electric units.

## Description

### CROSS-REFERENCE TO RELATED APPLICATIONS

This application claims priority to Chinese patent applications No. 202221564181.7, filed on June 21, 2022, titled "HEAT DISSIPATION SYSTEM OF RACK AND MULTI-MODE IMAGING DEVICE", No. 202310450102.2, filed on April 24, 2023, titled "COOLING APPARATUS OF MEDICAL DEVICE AND MEDICAL SYSTEM", No. 202320950600.9, filed on April 24, 2023, titled "COOLING APPARATUS OF MEDICAL DEVICE AND MEDICAL SYSTEM". The contents of the above applications are hereby incorporated herein in their entireties by reference.

### TECHNICAL FIELD

The present invention generally relates to the field of an image device, and in particular, to a heat dissipation system and a medical system.

### BACKGROUND

A multi-mode medical system is a novel imaging device that organically combines devices such as Positron Emission Tomography (PET), Computed Tomography (CT), Magnetic Resonance (MR) imaging, radiotherapy (RT), and Ultrasound (US) diagnostics.

An entire cooling solution of the multi-mode medical system is usually air-cooled with an air-cooled compressor, or water-cooled to dissipate heat of one of devices of the multi-mode medical system. In a high-end device, a requirement of a detector module on an ambient temperature is relatively high, so that a conventional air-cooled heat dissipation manner cannot implement a requirement that the ambient temperature is less than a few degrees. That is, a conventional heat dissipation manner applied to the multi-mode medical system cannot meet a requirement of high image quality in modem medicine.

### SUMMARY

According to various embodiments of the present invention, a heat dissipation system and a medical system are provided.

In a first aspect, a heat dissipation system configured for a medical system includes a circulation pipeline and a cooling device. The circulation pipeline is configured for conveying a cooling medium to the medical system, the medical system includes at least two electric units, and the at least two electric units have different rated cooling temperatures. The cooling device is in communication with the circulation pipeline and is configured for adjusting a temperature of the cooling medium, so as to dissipate heat of the at least two electric units.

In some embodiments, the at least two electric units include at least two medical devices, which include a first medical device and a second medical device.

In some embodiments, a rated cooling temperature of the first medical device is lower than that of the second medical device, the second medical device is connected to the first medical device in series by the circulation pipeline, so that at least a part of the cooling medium flowing out from the first medical device flows into the second medical device.

In some embodiments, the first medical device is in communication with the second medical device and the cooling device by the circulation pipeline, respectively, so that a part of the cooling medium flowing out from the first medical device flows into the second medical device, and the other of the cooling medium directly flows back to the cooling device.

In some embodiments, a flow adjusting apparatus is disposed in the circulation pipeline, and is configured to adjust a flow ratio of a part of the cooling medium which flows into the second medical device to a part of the cooling medium which directly flows back to the cooling device.

In some embodiments, the first medical device is provided with a first flow path, the second medical device is provided with a second flow path, and the first flow path and the second flow path are in communication with the cooling device in series by the circulation pipeline.

In some embodiments, the circulation pipe includes a first liquid outlet pipe, a first branch pipe, and a first return pipe, a liquid inlet end of the first flow path is in communication with the first liquid outlet pipe, a liquid outlet end of the first flow path is in communication with a liquid inlet end of the second flow path by the first branch pipe, and a liquid outlet end of the second flow path is in communication with the first return pipe.

In some embodiments, the circulation pipeline further includes a second branch pipe, and the second branch pipe and the second flow path are connected in parallel between the first flow path and the cooling device, or are connected in parallel between the first flow path and the first return pipe.

In some embodiments, the second medical device is connected to the first medical device in parallel by the circulation pipeline, so that the cooling medium flows into the first medical device and the second medical device, respectively.

In some embodiments, the first medical device is provided with a first flow path, the second medical device is provided with a second flow path, and the first flow path and the second flow path are in communication with the cooling device in parallel by the circulation pipeline.

In some embodiments, the circulation pipeline includes a second liquid outlet pipe and a second return pipe, a liquid inlet end of the first flow path and a liquid inlet end of the second flow path are in communication with the second liquid outlet pipe in parallel, and a liquid outlet end of the first flow path and a liquid outlet end of the second flow path are in communication with the second return pipe in parallel.

In some embodiments, the circulation pipeline includes a third liquid outlet pipe, a fourth liquid outlet pipe, a third return pipe, and a fourth return pipe, the first flow path is in communication with the cooling device by the third liquid outlet pipe and the third return pipe, and the second flow path is in communication with the cooling device by the fourth liquid outlet pipe and the fourth return pipe.

In some embodiments, the at least two medical devices include a third medical device, and both a rated cooling temperature of the first medical device and a rated cooling temperature of the second medical device are greater than that of the third medical device. The third medical device is connected to the first medical device and the second medical device in series by the circulation pipeline, the third medical device is disposed in front of the first medical device and the second medical device, so that at least a part of the cooling medium flows out from the third medical device flows into the first medical device and the second medical device.

In some embodiments, the at least two medical devices include a fourth medical device, and both a rated cooling temperature of the first medical device and a rated cooling temperature of the second medical device are less than that of the fourth medical device. The fourth medical device is connected to the first medical device and the second medical device in series by the circulation pipeline, the fourth medical device is disposed beyond the first medical device and the second medical device, so that at least a part of the cooling medium flows out from the first medical device and the second medical device flows into the fourth medical device.

In some embodiments, the first flow path includes a plurality of first branch flow paths which are disposed in parallel in the first medical device.

In some embodiments, the second flow path includes a plurality of second branch flow paths which are disposed in parallel in the second medical device.

In some embodiments, in the at least two medical devices, a medical device with a low rated cooling temperature is disposed in front of a medical device with a high rated cooling temperature, so that the cooling medium always flows from the medical device with the low rated cooling temperature into the medical device with the high rated cooling temperature.

In some embodiments, the at least two electric units include at least two load members of a same medical device, and the at least two load members include a first member and a second member.

In some embodiments, the circulation pipeline is connected to the first member and the second member in series, and the circulation pipeline is capable of conveying the cooling medium to the first member and the second member, respectively, wherein a rated cooling temperature of the first member is less than a rated cooling temperature of the second member.

In a second aspect, a medical system includes the heat dissipation system and the at least two electric units.

In some embodiments, the at least two electric units include at least two medical devices, which include at least one of a Computed Tomography device, a Magnetic Resonance imaging device, a Positron Emission Tomography device, a radiotherapy device, an Ultrasound diagnostics device, a Single-Photon Emission Computed Tomography device, or an X-ray imaging device, respectively.

### BRIEF DESCRIPTION OF THE DRAWINGS

In order to more clearly illustrate the technical solutions in the embodiments of the present invention or the related technology, the accompanying drawings to be used in the description of the embodiments or the related technology will be briefly introduced below, and it will be obvious that the accompanying drawings in the following description are only some of the embodiments of the present invention, and that, for one skilled in the art, other accompanying drawings can be obtained based on these accompanying drawings without putting in creative labor.
FIG. 1 is a schematic diagram of a heat dissipation system in an embodiment of the present invention.
FIG. 2 is a schematic diagram of a heat dissipation system in another embodiment of the present invention.
FIG. 3 is a schematic diagram of a heat dissipation system in another embodiment of the present invention.
FIG. 4 is a schematic diagram of a heat dissipation system in another embodiment of the present invention.
FIG. 5 is a schematic diagram of a first medical device in an embodiment of the present invention.
FIG. 6 is a schematic diagram of a second medical device in an embodiment of the present invention.
FIG. 7 is a schematic diagram of a heat dissipation system in another embodiment of the present invention.
FIG. 8 is a schematic diagram of a heat dissipation system in another embodiment of the present invention.
FIG. 9 is a schematic diagram of a heat dissipation system in another embodiment of the present invention.
FIG. 10 is a schematic diagram of a heat dissipation system in another embodiment of the present invention.
FIG. 11 is a schematic diagram of a heat dissipation system in another embodiment of the present invention.
FIG. 12 is a schematic diagram of a heat dissipation system in another embodiment of the present invention.
FIG. 13 is a schematic diagram of a heat dissipation system in another embodiment of the present invention.
FIG. 14 is a schematic diagram of a heat dissipation system in another embodiment of the present invention.
FIG. 15 is a schematic diagram of a heat dissipation system in another embodiment of the present invention.
FIG. 16 is a schematic diagram of a heat dissipation system in another embodiment of the present invention.
FIG. 17 is a schematic diagram of a heat dissipation system in another embodiment of the present invention.
FIG. 18 is a schematic diagram of a heat dissipation system in another embodiment of the present invention.
FIG. 19 is a schematic diagram of a heat dissipation system in another embodiment of the present invention.
FIG. 20 is a schematic diagram of a heat dissipation system in another embodiment of the present invention.
FIG. 21 is a schematic diagram of a heat dissipation system in another embodiment of the present invention.
FIG. 22 is a schematic diagram of a heat dissipation system in another embodiment of the present invention.
FIG. 23 is a schematic diagram of a heat dissipation system in another embodiment of the present invention.
FIG. 24 is a schematic diagram of assembling a heat dissipation system and a rotating rack in an embodiment of the present invention.
FIG. 25 is a schematic diagram of assembling a heat dissipation system and a rotating rack in another embodiment of the present invention.
FIG. 26 is a schematic diagram of a heat dissipation system in another embodiment of the present invention.
FIG. 27 is a schematic diagram of a medical system in an embodiment of the present invention.

### DETAILED DESCRIPTION OF THE EMBODIMENT

The following clearly and completely describes the technical solutions in the embodiments of the present invention with reference to the accompanying drawings in the embodiments of the present invention. Apparently, the described embodiments are merely some rather than all of the embodiments of the present invention. Based on the embodiments of the present invention, all other embodiments obtained by one skilled in the art without creative efforts fall within the protection scope of the present invention.

It should be noted that when an element is referred to be "set on" another element, it may be directly located on another element or may have a centered element. When an element is considered to be "disposed on" another element, it may be disposed directly on another element or may have a centered element at the same time. When an element is considered to be "fixed" to another element, it may be fixed directly to another element or may have a centered element at the same time.

The terms "first" and "second" are used only for the purpose of description and are not to be understood as indicating or implying relative importance or implying the number of indicated technical features. Therefore, a feature defined with "first" and "second" may explicitly or implicitly include at least one feature. In the description of the present invention, "a plurality of" means at least two, such as two and three, unless otherwise specifically limited.

Unless otherwise defined, all technical and scientific terms used herein are of the same meaning as generally understood by one skilled in the art. The terms used in the description of the present invention are merely intended to describe specific implementation manners, and are not intended to limit the present invention. The term "and/or" as used herein includes any and all combinations of one or more associated listed items.

A medical device, such as a Computer Tomography device, a Magnetic Resonance imaging device, a Positron Emission Tomography device, a radiotherapy device, an Ultrasound diagnostics device, a Single-Photon Emission Computed Tomography device, or an X-ray imaging device, whose load member, such as a detector module, is prone to heat due to power-on during operation. Due to heat accumulation, an actual temperature in operation of the medical device may exceed a certain limit, i.e., a rated cooling temperature. In addition, different types of medical devices may have different rated cooling temperatures, for example, PET typically needs to be in operation at a lower rated cooling temperature than both CT and MR. A heat dissipation system in an embodiment of the present invention is configured to perform heat dissipation on a heat source in the medical device, such as the detector module, so that the medical device meets a requirement of the rated cooling temperature. In the present invention, the rated cooling temperature may refer to a temperature at which the medical device is in operation normally.

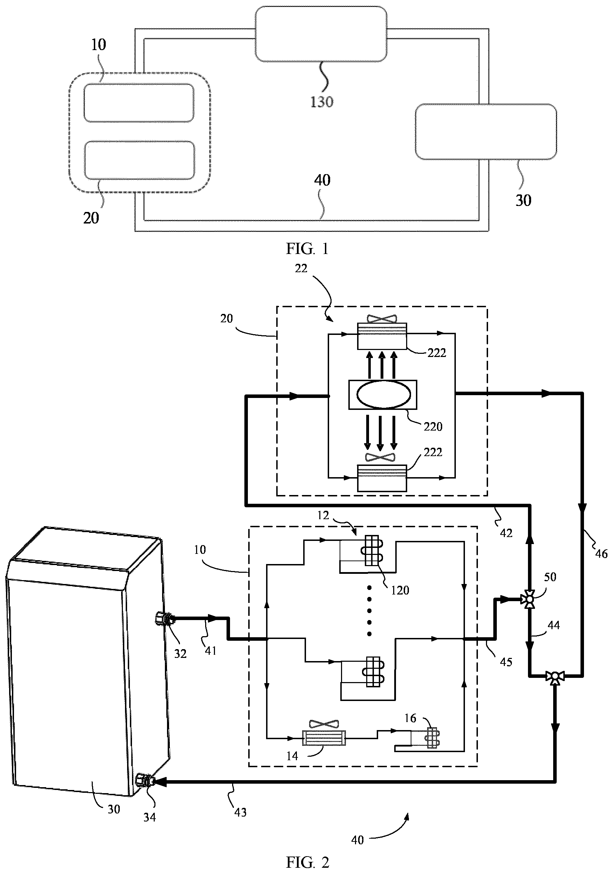

Referring to FIG. 1, a heat dissipation system is provided in an embodiment of the present invention, including a cooling device 30 and a circulation pipeline 40. The circulation pipeline 40 is configured for conveying a cooling medium to a medical system. The medical system includes at least two electric units, and the at least two electric units have different rated cooling temperatures. The cooling device 30 is in communication with the circulation pipeline 40 and is configured for adjusting a temperature of the cooling medium, so as to dissipate heat of the at least two electric units.

Referring to FIG. 2, in some embodiments, the circulation pipeline 40 may include a liquid outlet pipe and a return pipe. The liquid outlet pipe and the return pipe may be connected to an outlet port 32 and an inlet port 34 of the cooling device 30, respectively, to form a cooling medium circuit, so that the cooling medium obtained after heat dissipation is performed on the at least two electric units may return to the cooling device 30.

The cooling medium may be cooled in the cooling device 30, and heat exchange may occur between the cooling medium and the at least two electric units, thereby the cooling medium being heat up. Heat dissipation may be performed on the at least two electric units by a circulation of the cooling medium between the at least two electric units and the cooling device 30. In some embodiments, the cooling medium may be a gas, a liquid, or a combination thereof, for example, may include but is not limited to any one or a combination of coolants such as water, Freon, alcohol coolant, glycerol coolant, ethylene glycol coolant, and electronic fluorination liquid. In some embodiments, a cooling medium with insulation performance may be used to reduce a risk of a short circuit to a system card resulting from leak of the cooling medium.

The cooling device 30 may be configured to increase or decrease a temperature of the cooling medium in the circulation pipeline 40. For example, when the temperature of the cooling medium is higher than a preset threshold, the cooling device 30 may cool the cooling medium. When the temp erature of the cooling medium is lower than the preset threshold, the cooling device 30 may heat the cooling medium. In some embodiments, the cooling device 30 may include one or more heat exchange modules configured to exchange heat with the cooling medium, thereby adjusting the temperature of the cooling medium in the circulation pipeline 40.

The at least two electric units may include at least two medical devices, or at least two load members of a same medical device. The at least two medical devices may have different rated cooling temperatures, or the at least two load members of the same medical device may have different rated cooling temperatures. After heat exchange with the cooling device 30 and setting of the circulation pipeline 40, the temperature of the cooling medium may be equal to or lower than the rated cooling temperature of each of the at least two electric units when reaching a corresponding electric unit, so that temperatures of the at least two electric units may be kept at the rated cooling temperature, respectively, by heat exchange between the cooling medium and the at least two electric units.

In some embodiments, the at least two medical devices may include a first medical device 10 and a second medical device 20.

In some embodiments, the first medical device 10 and the second medical device 20 may be different imaging devices. For example, the first medical device 10 may be a PET device, and the second medical device 20 may be a CT device. In some embodiments, the first medical device 10 and the first medical device 20 may include an imaging device and a treatment device. For example, the first medical device 10 may include at least one of a PET device, a CT device, or an RT device, and the second medical device 20 may include at least one of an MR imaging device or a US diagnostic device.

In some embodiments, the first medical device 10 may include a first rack 12. The second medical device 20 may include a second rack 22. The first rack 12 is configured to carry a load member of the first medical device. The second rack 22 is configured to carry a load member of the second medical device.

In some embodiments, the first rack 12 may be connected to the second rack 22, for example, the first rack 12 and the second rack 22 may be an integrated structure, so that a structure of the heat dissipation system is more compact, space occupation is reduced, and a miniaturization requirement of a medical system is met.

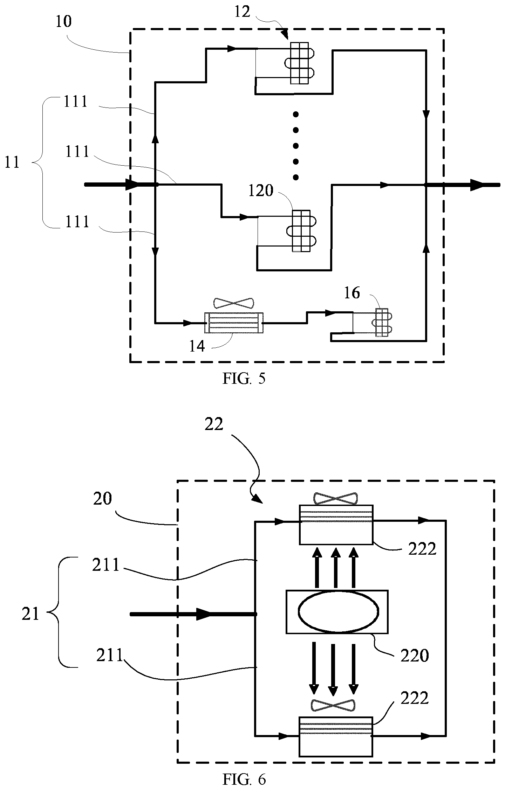

Referring to FIG. 5 and FIG. 6, in some embodiments, the first rack 12 may be provided with a first flow path 11, and the second medical device 22 may be provided with a second flow path 21. The cooling device 30 may import the cooling medium into the first flow path 11 and the second flow path 21 by the circulation pipeline 40, so as to dissipate heat of the load member on the first rack 12 and the load member on the second rack 22. The load member may generate heat due to power-on in operation, thus becoming a heating element. When the heat dissipation system is in operation, heat exchange may be performed between the cooling medium that flows through the first flow path 11 and the second flow path 21 and the load member, such as a detector module 220, to achieve a purpose of heat dissipation on the load member. In an embodiment, the cooling device 30 may specifically include a water cooler, and the cooling medium may include water-cooling liquid, so that when the water cooler is in operation, the water-cooling liquid may flow through the circulation pipeline 40, the first flow path 11 of the first medical device 10, and the second flow path 21 of the second medical device 20.

When the heat dissipation system is in operation, a heat dissipation effect on the medical device may be specifically controlled by controlling, according to a requirement of a rated cooling temperature of the medical device, a flow volume and a flow speed of the cooling medium that is imported into the first flow path 11 and the second flow path 21.

The heat dissipation system in the present embodiment of the present invention may perform simultaneous or alternating heat dissipation on the at least two medical devices in a heat exchange manner by the cooling medium, so as to improve heat dissipation efficiency of the heat dissipation system and reduce heat dissipation costs, and also reduce noise when a medical system is in operation to which the heat dissipation system is applied, improve user experience, and reduce a probability that foreign objects such as dust enter inside the heat dissipation system, so that the heat dissipation system in the present invention may be applicable to more high-end medical systems. The foregoing simultaneous heat dissipation may specifically refer to at least partial overlapping of heat dissipation of the at least two medical devices in time, for example, heat dissipation may be performed simultaneous at a same time, and alternating heat dissipation may specifically refer to alternating heat dissipation of the at least two medical devices in time.

In some embodiments, for example, in an embodiment referring to FIG. 2, the second medical device 20 may be connected to the first medical device 10 in series by the circulation pipeline 40. In some embodiments, a rated cooling temperature of the first medical device 10 may be lower than that of the second medical device 20. The first medical device 10 may be disposed in front of the second medical device 20, so that at least a part of the cooling medium flowing out from the first medical device 10 flows into the second medical device 20.

Since the rated cooling temperature of the first medical device 10 is lower than that of the second medical device 20, the cooling medium may preferentially meet heat dissipation on the first medical device 10, and first flow into the first rack 12 by the circulation pipeline 40, so as to exchange heat with the first medical device 10. While the heat dissipation is performed on the first medical device 10, the temperature of the cooling medium may rise. However, since the rated cooling temperature of the first medical device 10 is lower than that of the second medical device 20, the temperature of the cooling medium in this case may be still lower than the rated cooling temperature of the second medical device 20, the cooling medium may be further configured to perform heat dissipation on the second medical device 20, and the cooling medium with one temperature may need to be output from the cooling device 30 to meet a requirement for heat dissipation of the first medical device 10 and the second medical device 20 that have different rated cooling temperatures. At least a part of the cooling medium flowing out from the first rack 12 may flow into the second rack 22 by the circulation pipeline 40, so as to exchange heat with the second medical device 20 and perform heat dissipation on the second medical device 20. The cooling medium flowing out from the second rack 22 may return to the cooling device 30 to complete a cycle.

It may be understood that, to avoid that the medical device is damaged due to a large difference between a temperature of the circulation pipeline 40 and the rated cooling temperature of the medical device corresponding to the circulation pipeline 40, the temperature of the cooling medium in the circulation pipeline 40 should not be excessively low. As an example only, in some embodiments, the difference between the temperature of the cooling medium of the circulation line 40 and the rated cooling temperature of the corresponding medical device may be in a range of 5°C to 25°C.

In some embodiments, the first flow path 11 and the second flow path 21 may be in communication with the cooling device 30 in series by the circulation pipeline 40. For example, the first medical device 10 may be a PET device, and the second medical device 20 may be a CT device or an MR device. When the temperature of the cooling medium required for the PET device in operation, i.e., the rated cooling temperature, is lower than that required for the CT device or an MR device in operation, for example, the temperature of the cooling medium required for the first medical device 10 is lower than that required for the second medical device 20, the heat dissipation system in the present invention may first import the cooling medium into the first flow path 11 of the first rack 12, so as to meet a cooling medium with a low temperature required for the first medical device 10, and implement heat dissipation on the PET device used as the first medical device 10. The cooling medium that passes through the first rack 12 may absorb heat of the PET device, and the temperature of the cooling medium may rise. In this case, the temperature of the cooling medium may still meet a requirement of the CT device or the MR device that is used as the second medical device 20 for the temperature of the cooling medium, the cooling medium that is exported by the first rack 12 may be directly imported into the second flow path 21 of the second rack 22, so as to implement heat dissipation of the CT medical device or the MR medical device that is used as the second medical device 20.

In some embodiments, the circulation pipeline 40 may include a first liquid outlet pipe 41 and a first return pipe 43. Both ends of the first liquid outlet pipe 41 may be in communication with the outlet port 32 of the cooling device 30 and a liquid inlet end of the first flow path 11, respectively. The cooling medium may flow out from the cooling device 30, and enter the first flow path 11 through the first liquid outlet pipe 41. The cooling medium may flow out from the first flow path 11 and enter the second flow path 21. Both ends of the first return pipe 43 may be in communication with a liquid inlet port 34 of the cooling device 30 and a liquid outlet end of the second flow path 21, respectively. The cooling medium may flow out from the second flow path 21 and flow back to the cooling device 30 through the first return pipe 43.

In some embodiments, to enable the second medical device 20 to operate in a proper temperature range, the cooling medium flowing out from the first medical device 10 may not need or not be suitable for all flowing into the second medical device 20, and the first medical device 10 may be in communication with the second medical device 20 and the cooling device 30 by the circulation pipeline 40, respectively, so that a part of the cooling medium flowing out from the first medical device 10 may flow into the second medical device 20, and the other part of the cooling medium may be in short circuit with the second medical device 20 and directly flow back to the cooling device 30.

In some embodiments, the circulation pipeline 40 may further include at least two branch pipes, such as a first branch pipe 42 and a second branch pipe 44. The first branch pipe 42 and the second branch pipe 44 may be in communication with a liquid outlet end of the first flow path 11, respectively. Both ends of the first branch pipe 42 may be in communication with the liquid outlet end of the first flow path 11 and a liquid inlet end of the second flow path 21, respectively. Both ends of the second branch pipe 44 may be in communication with the liquid outlet end of the first flow path 11 and a liquid inlet end of the first return pipe 43, respectively. The second branch pipe 44 and the second flow path 21 may be connected in parallel between the first flow path 11 and the cooling device 30, or specifically connected in parallel between the first flow path 11 and the first return pipe 43.

In some embodiments, the circulation pipeline 40 may further include a first infusion pipe 45 and a second infusion pipe 46. A liquid inlet end of the first infusion pipe 45 may be in communication with the liquid outlet end of the first flow path 11. The first branch pipe 42 and the second branch pipe 44 may be in communication with a liquid outlet end of the first infusion pipe 45, respectively. Both ends of the second infusion pipe 46 may be in communication with the liquid outlet end of the second flow path 21 and the liquid inlet end of the first return pipe 43, respectively.

In the present embodiment, when the heat dissipation system is in operation, the cooling medium in the cooling device 30 may flow into the first flow path 11 from the first liquid outlet pipe 41, and the cooling medium flowing out from the first flow path 11 may be divided into at least two parts. A part of the cooling medium may flow into the second flow path 21 through the first branch pipe 42, and enter the first return pipe 43 after flowing out from the second flow path 21. The other part of the cooling medium may not enter the second flow path 21, but directly enter the first return pipe 43 through the second branch pipe 44. At the liquid inlet end of the first return pipe 43, the part of the cooling medium that enters the second flow path 21 may be combined with the other part of the cooling medium that does not enter the second flow path 21.

In some embodiments, a flow adjusting apparatus 50, such as a valve, may be disposed in the circulation pipeline 40 to adjust a flow ratio of a part of the cooling medium which flows into the second medical device 20 to a part of the cooling medium which is in short circuit with the second medical device 20 and directly flows back to the cooling device 30. The flow adjusting apparatus 50 may easily control flow of the cooling medium flowing into the second medical device 20, so that heat dissipation capacity of the second medical device 22 may be more easily and accurately controlled, and the second medical device 22 may be more easily in operation in a proper temperature range.

In some embodiments, the first branch pipe 42 and the second branch pipe 44 may be in communication with the liquid outlet end of the first flow path 11 by the flow adjusting apparatus 50, respectively. Specifically, the first branch pipe 42 and the second branch pipe 44 may be in communication with the liquid outlet end of the first infusion pipe 45 by the flow adjusting apparatus 50, respectively.

In some embodiments, another flow adjusting apparatus 50, such as another valve, may be disposed in the circulation pipeline 40 to combine the part of the cooling medium that enters the second flow path 21 with the part of the cooling medium that does not enter the second flow path 21. Specifically, a liquid outlet end of the second branch pipe 44 and a liquid outlet end of the second infusion pipe 46 may be in communication with the liquid inlet end of the first branch pipe 43 by the another flow adjusting apparatus 50, respectively.

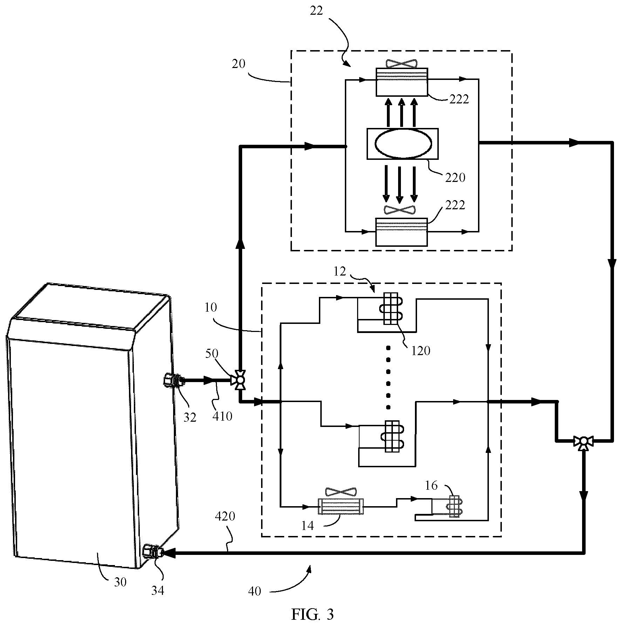

Referring to FIG. 3, in some embodiments, the second medical device 20 and the first medical device 10 may be connected in parallel by the circulation pipeline, so that the cooling medium may flow into the first medical device 10 and the second medical device 20, respectively. Specifically, the first flow path 11 and the second flow path 21 may be in communication with the cooling device 30 in parallel by the circulation pipeline 40.

In the present embodiment, the circulation pipeline 40 may include a second liquid outlet pipe 410 and a second return pipe 420. A liquid inlet end of the first flow path 11 and a liquid inlet end of the second flow path 21 may be in communication with the second liquid outlet pipe 410 in parallel, and a liquid outlet end of the first flow path 11 and a liquid outlet end of the second flow path 21 may be in communication with the second return pipe 420 in parallel. The cooling medium may flow out from the cooling device 30, after passing through the second liquid outlet pipe 410, a part of the cooling medium may enter the first flow path 11, and the other part of the cooling medium may enter the second flow path 21. The cooling medium that passes through the first flow path 11 and the second flow path 21 respectively may converge into the second return pipe 420. In other words, the first medical device 10 and the second medical device 20 in the present embodiment may be specifically in communication between the second liquid outlet pipe 410 and the second return pipe 420.

In some embodiments, the circulation pipeline 40 may include a flow adjusting apparatus 50 and a branch pipe. The second liquid outlet pipe 410 may be in communication with two branch pipes respectively by the flow adjusting apparatus 50, such as a valve, and be in communication with the liquid inlet end of the first flow path 11 and the liquid inlet end of the second flow path 21 by the two branch pipes, respectively. The second return pipe 420 may be in communication with the two branch pipes respectively by another flow adjusting apparatus 50, such as another valve, and be in communication with the liquid outlet end of the first flow path 11 and the liquid outlet end of the second flow path 21 by the two branch pipes, respectively. The flow adjusting apparatus 50 may easily control the flow ratio of the cooling medium flowing into the first medical device 10 to that flowing into the second medical device 20, so that the heat dissipation capacities of the first medical device 12 and the second medical device 22 may be more easily and accurately controlled, and therefore, the first medical device 12 and the second medical device 22 may be more easily in operation in a proper temperature range.

In the present embodiment, when the heat dissipation system in operation, the cooling medium in the cooling device 30 may be imported into the first flow path 11 and the second flow path 21, respectively, after being exported from the second liquid outlet pipe 410, and then the cooling medium passing through the first medical device 10 and the second medical device 20 may return to the cooling device 30 by the second return pipe 420. The cooling medium that returns to the cooling device 30 may be cooled by the cooling device 30, and then flow out to the second liquid outlet pipe 410 again, thereby implementing a cycle of the cooling medium.

It may be understood that, when the first flow path 11 is connected to the second flow path 21 in parallel, the first flow path 11 of the first rack 12 and the second flow path 21 of the second rack 22 may be connected in parallel between the second liquid outlet pipe 410 and the second return pipe 420, which is not limited herein. In an embodiment, the first flow path 11 and the second flow path 21 may be directly in communication with the cooling device 30.

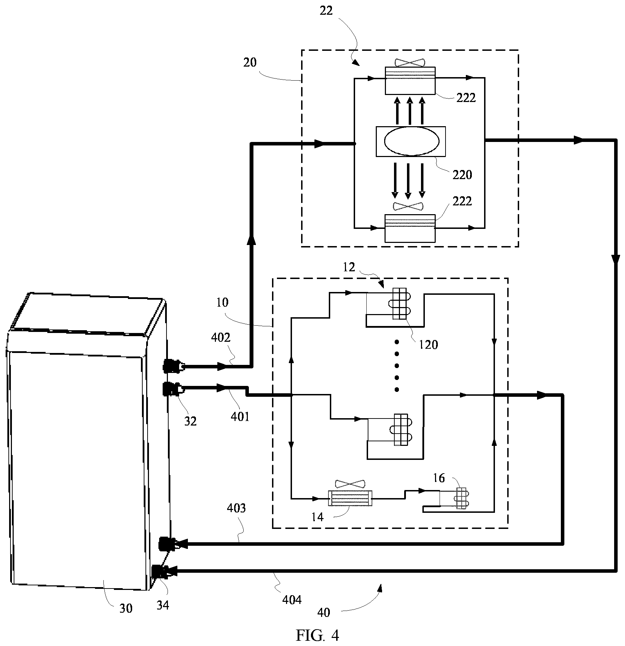

Referring to FIG. 4, in some embodiments, the circulation pipeline 40 may include a third liquid outlet pipe 401, a fourth liquid outlet pipe 402, a third return pipe 403, and a fourth return pipe 404. The first flow path 11 may be in communication with the cooling device 30 by the third liquid outlet pipe 401 and the third return pipe 403, and the second flow path 21 may be in communication with the cooling device 30 by the fourth liquid outlet pipe 402 and the fourth return pipe 404. Specifically, the liquid inlet end of the first flow path 11 may be in communication with the third liquid outlet pipe 401, and the liquid outlet end of the first flow path 11 may be in communication with the third return pipe 403. The liquid inlet end of the second flow path 21 may be in communication with the fourth liquid outlet pipe 402, and the liquid outlet end of the second flow path 21 may be in communication with the fourth return pipe 404. Outside the cooling device 30, the cooling medium may be divided into at least two completely independent parts, which flow through the first flow path 11 and the second flow path 21, respectively. Different parts of the cooling medium may flow together or still be independent of each other after entering the cooling device 30, which is not limited in the present invention.

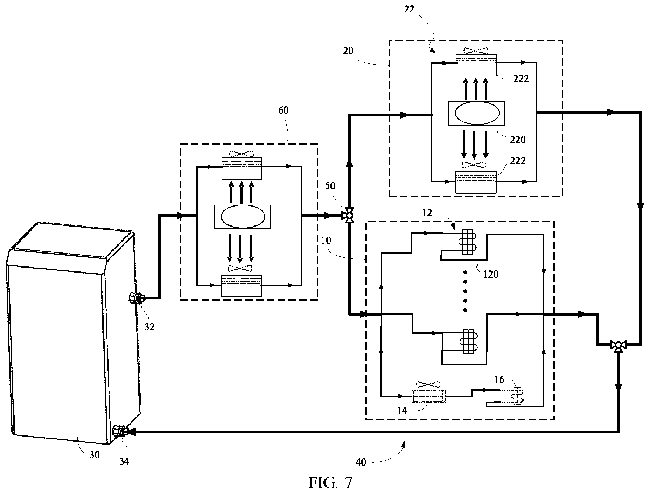

Referring to FIG. 7, in some embodiments, the at least two medical devices may further include a third medical device 60. There is a rated cooling temperature difference among the third medical device 60, the first medical device 10 and the second medical device 20.

In an embodiment, the first medical device 10 may be a PET device, the second medical device 20 may be a CT device, and the third medical device 60 may be an MR device.

In some embodiments, a third flow path may be disposed in the third medical device 60. The cooling device 30 may be in communication with the third medical device 60 by the circulation pipeline 40, and the cooling device 30 may import the cooling medium into the third flow path by the circulation pipeline 40, so as to perform heat dissipation on the third medical device 60.

In some embodiments, the first medical device 10, the second medical device 20, and the third medical device 60 may be connected with each other in series or parallel by the circulation pipeline 40. Alternatively, two medical devices of the first medical device 10, the second medical device 20, and the third medical device 60 may be connected with each other in parallel by the circulation pipeline 40, and then be connected to the other medical device in series by the circulation pipeline 40. Alternatively, two medical devices of the first medical device 10, the second medical device 20, and the third medical device 60 may be connected with each other in series by the circulation pipeline 40, and then be connected to the other medical device in parallel by the circulation pipeline 40.

In some embodiments, both a rated cooling temperature of the first medical device and a rated cooling temperature of the second medical device may be greater than that of the third medical device, and the third medical device 60 may be disposed in front of the first medical device 10 and the second medical device 20 along a flow direction of the cooling medium. For example, the third medical device 60 may be connected in series to the first medical device 10 and the parallel second medical device 20 in parallel by the circulation pipeline 40. The third medical device 60 may be disposed in front of the first medical device 10 and the second medical device 20 in parallel, so that at least a part of the cooling medium flowing out from the third medical device 60 may flow into the first medical device 10 and the second medical device 20 in parallel.

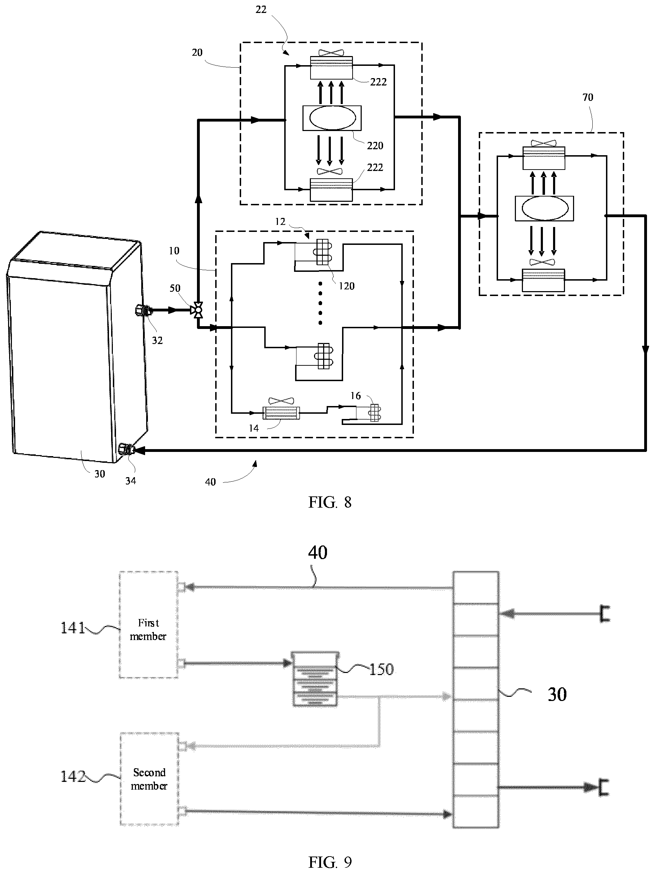

Referring to FIG. 8, in some embodiments, the at least two medical devices may further include a fourth medical device 70. Both the rated cooling temperatures of the first medical device 10 and the rated cooling temperatures of the second medical device 20 may be less than that of the fourth medical device 70, so that the fourth medical device 70 may be disposed beyond the first medical device 10 and the second medical device 20 along the flow direction of the cooling medium. For example, the fourth medical device 70 may be connected in series to the first medical device 10 and the parallel second medical device 20 in parallel by the circulation pipeline, and the fourth medical device 70 may be disposed beyond the first medical device 10 and the parallel second medical device 20 in parallel, so that at least a part of the cooling medium flowing out from the first medical device 10 and the parallel second medical device 20 in parallel may flow into the fourth medical device 70.

Overall, along the flow direction of the cooling medium, a medical device with a low rated cooling temperature in the at least two medical devices may be disposed in front of a medical device with a high rated cooling temperature, so that the cooling medium may always flow from the medical device with the low rated cooling temperature into the medical device with the high rated cooling temperature. In this way, the cooling medium with a temperature that is output from the cooling device 30 may meet heat dissipation requirements of a plurality of medical devices with different rated cooling temperatures, improve heat dissipation efficiency of the heat dissipation system, reduce heat dissipation costs, reduce noise when a medical system that applies the heat dissipation system is in operation, improve user experience, and reduce a probability that foreign objects such as dust enters inside the heat dissipation system, so that the heat dissipation system in the present invention may be applicable to more high-end medical systems.

In some embodiments, the first flow path 11 may include a plurality of first branch flow paths 111. The plurality of first branch flow paths 111 may be disposed in parallel in the first rack 12, so that each of the first branch flow paths 111 may perform water-cooling heat dissipation on at least one load member on the first rack 12, such as a detector module 120, to meet a water-cooling heat dissipation requirement of the first medical device 10. In an embodiment, the first medical device 10 may be a PET device, and the first branch flow paths 111 may be in the first rack 12. Specifically, it may be set according to a heat dissipation requirement of the PET device that each of the first branch flow paths 111 is corresponding to one detector module 120, or a plurality of detector modules 120 at the same time.

In some embodiments, the heat dissipation system may further include a heat sink 14 and a heat sink 16 disposed on the first rack 12, which may be specifically different types of heat sinks, such as a fan or a heat conductive plate, so that the cooling medium in the first flow path 11 may be further cooled outside the cooling device 30.

In some embodiments, the second flow path 21 may include a plurality of second branch flow paths 211, and the plurality of second branch flow paths 211 may be disposed in parallel in the second rack 22, so that each of the second branch flow paths 211 may perform water-cooling heat dissipation on at least one detector module 220 on the second rack 22 to meet a water-cooling heat dissipation requirement of the second medical device 20. In an embodiment, the first medical device 10 may be a CT device or an MR device, and the plurality of second branch flow paths 211 may be in the second rack 22. Specifically, it may be set according to a heat dissipation requirement of the CT device or the MR device that each of the second branch flow paths 211 is corresponding to one detector module 120, or a plurality of detector modules 120 at the same time.

In some embodiments, the heat dissipation system may further include a heat sink 222 disposed on the second rack 22, such as a fan or a heat conductive plate, so that the cooling medium in the second flow path 21 may be further cooled outside the cooling device 30.

Referring to FIG. 9, in some embodiments, the at least two electric units may include at least two load members of a same medical device. The at least two load members may include a first member 141 and a second member 142.

In some embodiments, the circulation pipeline 40 may be connected to the first member 141 and the second member 142 in series, and the circulation pipeline 40 may sequentially deliver the cooling medium to the first member 141 and the second member 142. A rated cooling temperature of the first member 141 may be less than that of the second member 142.

In some embodiments, a cooling medium storage apparatus 150 may be disposed between the first member 141 and the second member 142, and the cooling medium storage apparatus 150 is configured to store or buffer the cooling medium flowing out from the first member 141. In some embodiments, the cooling medium storage apparatus 150 may include one or more water tanks.

The cooling medium storage apparatus 150 may be connected to the cooling device 30 and the second member 142. When heat dissipation does not need to be performed on the second member 142, a pipeline between the cooling medium storage apparatus 150 and the second member 142 may be in a closed state, and the cooling medium in the cooling medium storage apparatus 150 may directly flow back to the cooling device 30 for cooling. On the contrary, when heat dissipation needs to be performed on the second member 142, the pipeline between the cooling medium storage apparatus 150 and the second member 142 may be in a communicating state, and a part or all of the cooling medium in the cooling medium storage apparatus 150 may flow to the second member 142, so that the heat dissipation may be performed on the second member 142.

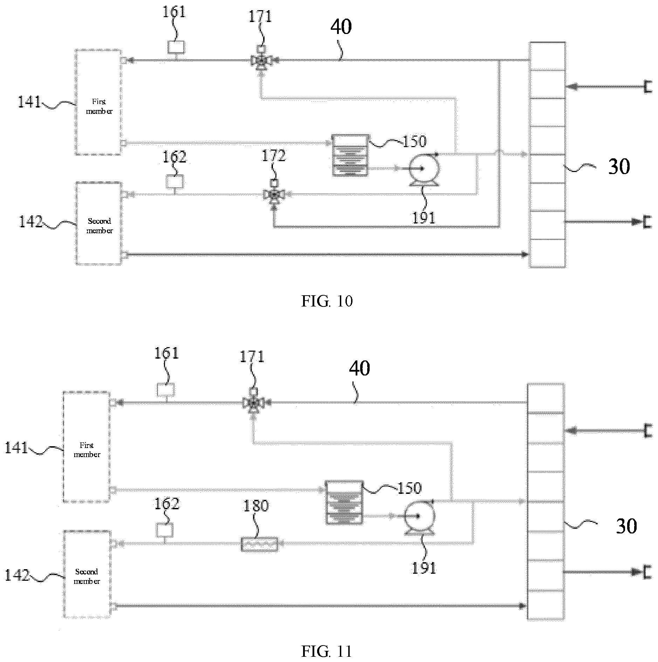

Referring to FIG. 10, in some embodiments, a temperature sensor 161 may be disposed at a cooling medium inlet end of the first member 141 to collect a temperature of the cooling medium flowing into the first member 141, and a temperature sensor 162 may be disposed at a cooling medium inlet end of the second member 142 to collect a temperature of the cooling medium flowing into the second member 142. An outlet end of the cooling medium storage apparatus 150 may be connected to a cooling medium inlet end of the first member 141 by a pipeline and a mixing valve 171. When the temperature sensor 161 collects the temperature of the cooling medium flowing into the first member 141 that is too low, the cooling medium in the cooling medium storage apparatus 150 may be recirculated to the first member 141 by the mixing valve 171. In some embodiments, a mixing ratio of the cooling medium flowing out from the cooling medium storage device 150 to that flowing out from the cooling device 30 may be controlled by controlling opening of each valve in the mixing valve 171 to meet a temperature requirement of the first member 141.

In some embodiments, the outlet end of the cooling device 30 may be connected to an inlet end of the second member 142 by a pipeline and a mixing valve 172. When the temperature sensor 162 collects the temperature of the cooling medium flowing into the second member 142 that is too high, the cooling medium flowing out from the cooling medium storage apparatus 150 may be mixed with the cooling medium coming from the cooling device 30, thereby reducing the temperature of the cooling medium flowing into the second member 142. Similarly, in some embodiments, a mixing ratio of the cooling medium flowing out from the cooling medium storage device 150 to that flowing out from the cooling device 30 may be controlled by controlling opening of each valve in the mixing valve 172 to meet a temperature requirement of the second member 142.

In some embodiments, a driving module 191 (e.g., a pump) may be provided at the outlet end of the cooling medium storage apparatus 150, thereby providing power to flow the cooling medium to the first member 141, the second member 142, or the cooling device 30 from the cooling medium storage apparatus 150.

Referring to FIG. 11, in some embodiments, a heating module 180 may be further disposed at a cooling medium inlet end of the second member 142. When the temperature sensor 162 collects the temperature of the cooling medium flowing into the second member 142 that is too low, the heating module 180 may heat up the cooling medium flowing into the second member 142, so as to meet a temperature requirement of the cooling medium flowing into the second member 142 better.

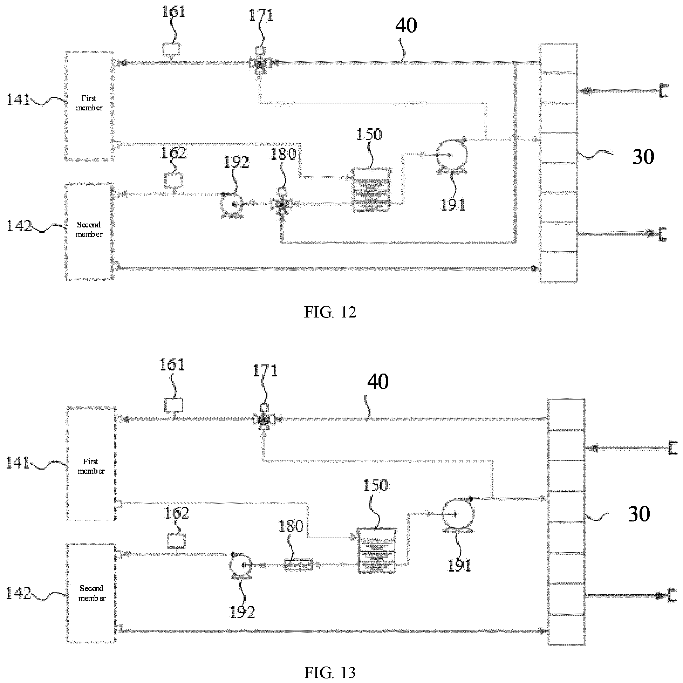

Refer to FIG. 12 and FIG. 13, in some embodiments, the cooling medium storage apparatus 150 may include two output ports. The cooling medium flowing out from one output port may flow to the first member 141 and/or the cooling device 30, and the cooling medium flowing out from the other output port may flow to the second member 142. In some embodiments, to provide power to the cooling medium for flowing to the first member 141, the second member 142, or the cooling device 30 better, a driving module may be disposed for the two output ports of the cooling medium storage apparatus 150, respectively. For example, a driving module 191 may be disposed between an output port of the cooling medium storage apparatus 150 and the first member 141 (or the cooling device 30), and a driving module 192 may be disposed between another output port of the cooling medium storage apparatus 150 and the second member 142.

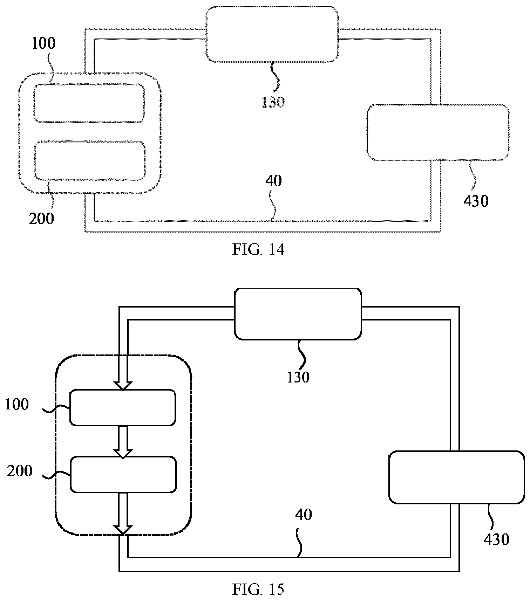

Referring to FIG. 14, in some embodiments, the at least two electric units may include a first electric unit 100 and a second electric unit 200. In some embodiments, the heat dissipation system may further include a first heat exchange module 430. The first heat exchange module 430 may be connected to the circulation pipeline 40, and configured to adjust the temperature of the cooling medium in the circulation pipeline 40.

In some embodiments, to accelerate a flow rate of the cooling medium in the circulation pipeline 40, and improve heat exchange efficiency of the heat dissipation system, the heat dissipation system may further include a first driving module 130. The first driving module 130 may be connected in the circulation pipeline 40, and configured to drive the cooling medium in the circulation pipeline 40 to flow to the first electric unit 100 and/or the second electric unit 200. In some embodiments, the first driving module 130 may include various types of driving pumps such as a gear pump, a blade pump, a plunger pump, or the like. In some embodiments, the first driving module 130 may be a shielded pump or a magnetic pump. In some embodiments, the cooling medium may be in a gaseous state, and the first driving module 130 may further be a fan.

In some embodiments, the first driving module 130 may be controlled manually or automatically. Exemplarily, when the first driving module 130 is manually controlled, a related worker may manually control the first driving module 130 to start when heat dissipation needs to be performed on the medical device. When the first driving module 130 is automatically controlled, a current working status and a heat dissipation requirement of the medical device may be detected based on a sensor (e.g., a temperature sensor) disposed at the medical device, and the first driving module 130 may be automatically controlled to be enabled or disabled based on the current operation status and the heat dissipation requirement of the medical device that is reflected by the data collected by the sensor. In some embodiments, a driving power of the first driving module 130 may be controlled to adjust the flow of the cooling medium flowing through the first electric unit 100 and the second electric unit 200.

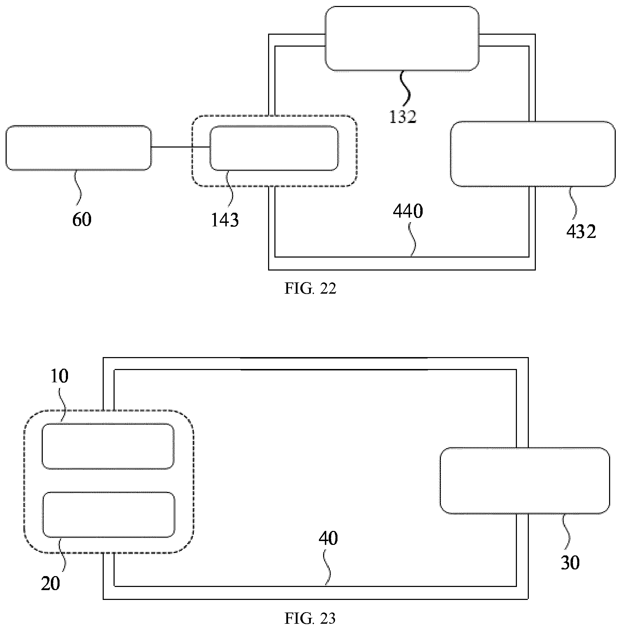

Referring to FIG. 23, in some embodiments, the heat dissipation system may not provide the first driving module 130. For example, convection may be formed based on a temperature difference of the cooling medium at different positions, so that the cooling medium in the circulation pipeline 40 circulates.

Referring to FIG. 15, in some embodiments, the circulation pipeline 40 may be connected in series to the first electric unit 100 and the second electric unit 200. Specifically, the cooling medium in the circulation pipeline 40 may sequentially flow through the first electric unit 100 and the second electric unit 200. In some embodiments, the first electric unit 100 and the second electric unit 200 may have the same or different rated cooling temperatures.

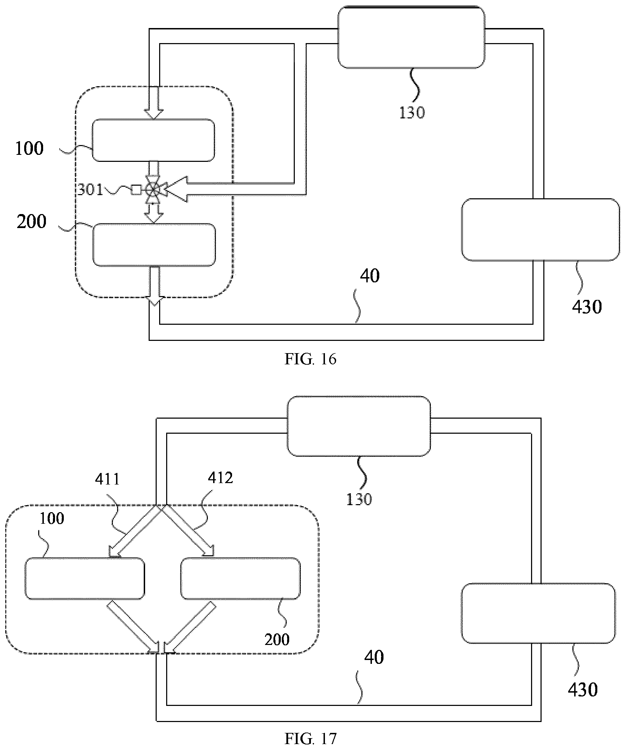

Referring to FIG. 16, in some embodiments, a three-way valve 301 may be disposed between the first electric unit 100 and the second electric unit 200. Two ports of the three-way valve 301 may be connected to an outlet end of the first electric unit 100 and an inlet end of the second electric unit 200, respectively, and a third port of the three-way valve 301 may be connected to a trunk line of the circulation pipeline 40 in front of an inlet end of the first electric unit 100 (e.g., a part between the inlet end of the first electric unit 100 and the first heat exchange module 430). In some embodiments, a control module may adjust the temperature of the cooling medium flowing into the second electric unit 200 by controlling a degree of opening and closing of the ports of the three-way valve 301.

In some embodiments, a temperature sensor may be disposed at an inlet of the second electric unit 200 to collect the temperature of the cooling medium flowing into the second electric unit 200. A control module may control, based on temperature data collected by the temperature sensor, a driving power of the first driving module 130, so as to ensure that the temperature of the cooling medium flowing into the second electric unit 200 meets a preset requirement by controlling the flow of the cooling medium in the circulation pipeline 40. In some embodiments, when there are a plurality of electric units, a temperature sensor may be disposed at an inlet of each of the plurality of electric units to collect the temperature of the cooling medium flowing into each of the plurality of electric unit.

In some embodiments, after the cooling medium flows out from the second electric unit 200, the cooling medium may be input to the first heat exchange module 430, and the cooling medium flowing out from the second electric unit 200 may be cooled by the first heat exchange module 430. In some embodiments, the first heat exchange module 430 may be connected to the cooling device 30, which may provide a cooling medium with a lower temperature (e.g., less than 20°C) for the first heat exchange module 430, and recover the cooling medium flowing out from the second electric unit 200. As an example only, in some embodiments, the cooling device 30 may include a condenser, which may be disposed outside, so that heat exchange may be performed with outdoor air, thereby dissipating heat in the outdoor air.

Referring to FIG. 17, in some embodiments, when a rated cooling temperature difference between the first electric unit 100 and the second electric unit 200 is not large (e.g., less than 5 °C), the circulation pipeline 40 may also be connected in parallel to the first electric unit 100 and the second electric unit 200. Specifically, the cooling medium in the circulation pipeline 40 may simultaneously flow to the first electric unit 100 and the second electric unit 200 at a same or approximately same temperature.

When the circulation pipeline 40 is connected in parallel to the first electric unit 100 and the second electric unit 200, the circulation pipeline 40 may include a first branch 411 and a second branch 412. The first branch 411 is configured to transport the cooling medium to the first electric unit 100, and the second branch 412 is configured to transport the cooling medium to the second electric unit 200. In some embodiments, a length and a diameter of the first branch 411 may be the same as or different from those of the second branch 412. For example, when heat generated by the first electric unit 100 in operation is greater than that generated by the second electric unit 200, the diameter of the first branch 411 may be greater than that of the second branch 412, so that in a case of a certain driving pressure, the flow of the cooling medium flowing to the first electric unit 100 in a unit time is greater than that flowing to the second electric unit 200. It should be noted that in this description, the diameter of the first branch 411 may refer to an average inner diameter of the first branch 411, the diameter of the second branch 412 may refer to an average inner diameter of the second branch 412.

In some embodiments, in order to control the flow of the cooling medium to the first electric unit 100 and/or the second electric unit 200, and further to control the temperature of the cooling medium to the first electric unit 100 and/or the second electric unit 200, a flow controller may be disposed on an inlet pipeline of the first branch 411 and/or the second branch 412. The inlet pipeline of the first branch 411 may be a part between the inlet end of the first electric unit 100 and the trunk line of the circulation pipeline 40 (e.g., a part between the first heat exchange module 430 and the first branch 411). The inlet pipeline of the second branch 412 may be a part between the inlet end of the second electric unit 200 and the trunk line of the circulation pipeline 40 (e.g., a part between the first heat exchange module 430 and the second branch 412).

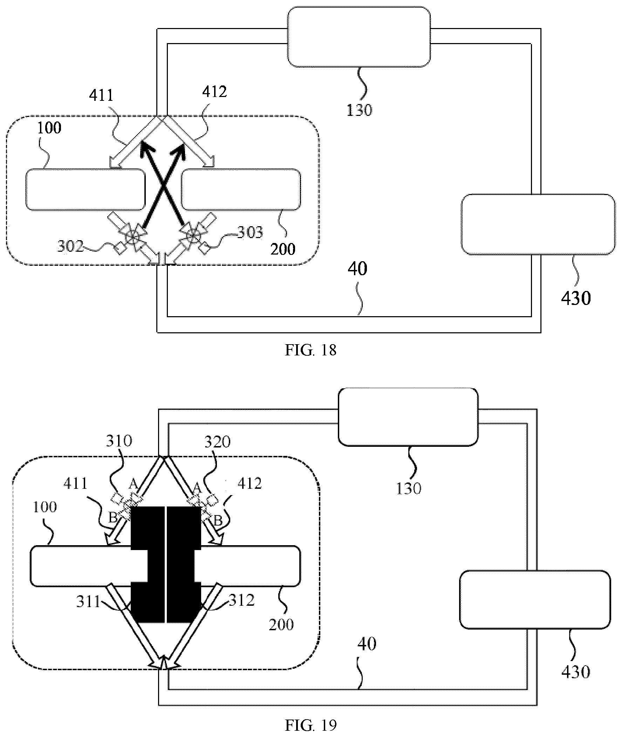

Referring to FIG. 18, in some embodiments, when the circulation pipeline 40 is connected in parallel to the first electric unit 100 and the second electric unit 200, a three-way valve 302 may be disposed at the outlet end of the first electric unit 100, and one port of the three-way valve 302 may be connected to the inlet end of the second electric unit 200, so that the cooling medium flowing out from the first electric unit 100 can flow to the second electric unit 200. Furthermore, a control module may adjust, by controlling a degree of opening and closing of each port of the three-way valve 302, the temperature of the cooling medium flowing into the second electric unit 200. Similarly, in some embodiments, a three-way valve 303 may be disposed at the outlet end of the second electric unit 200, and one port of the three-way valve 303 may be connected to the inlet end of the first load group 211, so that the cooling medium flowing out from the second electric unit 200 can be left to the first electric unit 100. Furthermore, a control module may adjust, by controlling a degree of opening and closing of each port of the three-way valve 303, the temperature of the cooling medium flowing into the first electric unit 100. In this way, even though a difference between the rated cooling temperature of the first electric unit 100 and the second electric unit 200 is relatively large (e.g., greater than 5 °C), the first electric unit 100 and the second electric unit 200 may be connected in parallel.

In some embodiments, it is considered that only a three-way valve 302 and a three-way valve 303 are disposed at the outlet end of the first electric unit 100 and at the outlet end of the second electric unit 200, a mixing ratio of the cooling medium flowing into the first electric unit 100 to that flowing into the second electric unit 200 may not be controlled well, thereby causing that the temperature of the cooling medium flowing into the first electric unit 100 and the second electric unit 200 cannot be accurately controlled. Therefore, to more accurately control the temperature of the cooling medium flowing into the first electric unit 100 and the second electric unit 200, in some embodiments, a three-way valve may be disposed at the inlet end of the first electric unit 100, and another three-way valve may be disposed at the inlet end of the second electric unit 200, so that one port of the three-way valve 303 may be connected to one port of the three-way valve disposed at the inlet end of the first electric unit 100, and one port of the three-way valve 302 may be connected to one port of the three-way valve disposed at the inlet end of the second electric unit 200. Furthermore, a control module may adjust, by controlling a degree of opening and closing of each port in the three-way valve 303 and the three-way valve disposed at the inlet end of the first electric unit 100, the temperature of the cooling medium flowing into the first electric unit 100, and adjust, by controlling a degree of opening and closing of each port in the three-way valve 302 and the three-way valve disposed at the inlet end of the second electric unit 200, the temperature of the cooling medium flowing into the second electric unit 200.

In some embodiments, a cooling apparatus may include another structure. For example, in some embodiments, the three-way valve 302 may also be connected to the inlet end of the first electric unit 100 or one port of the three-way valve disposed at the inlet end of the first electric unit 100. Similarly, in some embodiments, the three-way valve 303 may be connected to the inlet end of the second electric unit 200 or one port of the three-way valve disposed at the inlet end of the second electric unit 200.

Referring to FIG. 19, in some embodiments, a first flow controller 310 may be disposed in the inlet pipeline of the first branch 411. Similarly, a second flow controller 320 may be disposed in an inlet pipeline of the second branch 412. The first flow controller 310 and the second flow controller 320 may control flow of the cooling medium flowing to the first electric unit 100 and/or the second electric unit 200, so as to control the temperature of the cooling medium of the first electric unit 100 and/or the second electric unit 200.

In some embodiments, the first flow controller 310 or the second flow controller 320 may include a three-way valve, which may include three connecting ports A, B, and C. The port A of the first flow controller 310 may be connected to the first driving module 130, the port B of the first flow controller 310 may be connected to the inlet end of the first electric unit 100, and the port C of the first flow controller 310 may be connected to an outlet pipeline of the first branch 411. Similarly, the port A of the second flow controller 320 may be connected to the first driving module 130, the port B of the second flow controller 320 may be connected to the inlet end of the second electric unit 200, and the port C of the second flow controller 320 may be connected to an outlet pipeline of the second branch 412. It may be understood that, in this description, the outlet pipeline of the first branch 411 may be a part between the outlet end of the first electric unit 100 and the trunk line of the circulation pipeline 40 (e.g., a part between the first heat exchange module 430 and the first branch 411). The outlet pipeline of the second branch 412 may be a part between the outlet end of the second electric unit 200 and the trunk line of the circulation pipeline 40 (e.g., a part between the first heat exchange module 430 and the second branch 412).

In some embodiments, by conducting the outlet pipeline of the first branch 411 and/or the second branch 412 to a part of the flow controller (e.g., to the port C), the cooling medium flowing out from the outlet line of the first branch 411 may be imported into the inlet pipeline of the first branch 411 through an outlet branch 311, the cooling medium flowing out from the outlet line of the second branch 412 may be imported into the inlet pipeline of the second branch 412 through an outlet branch 312, so that a high temperature cooling medium flowing out of the outlet line of the first branch 411 may be imported into the inlet line of the first branch 411, a high temperature cooling medium flowing out of the outlet line of the second branch 412 may be imported into the inlet line of the second branch 412, so as to adjust the temperature of the cooling medium flowing out of the first branch 411 and/or the second branch 412. In addition, by the structure, cyclic use of the cooling medium may be implemented when the temperature of the cooling medium flowing out of the outlet pipeline of the first branch 411 and/or the second branch 412 meet reuse. Specifically, in some embodiments, a temperature sensor may be disposed on the outlet pipeline of the first branch 411 and/or the second branch 412 to collect the temperature of the cooling medium flowing out of the outlet pipeline of the first branch 411 and/or the second branch 412. When the temperature is less than a corresponding rated cooling temperature, a valve of the port C may be controlled to open, so that the cooling medium flowing out of the outlet pipeline of the first branch 411 may flow again from the inlet pipeline of the first branch 411 to the first electrical unit 100, and the cooling medium flowing out of the outlet pipeline of the second branch 412 may flow again from the inlet pipeline of the second branch 412 to the second electrical unit 200. In some embodiments, the temperature of the cooling medium in the first branch 411 may be controlled by controlling the opening of the ports A, B, and C of the first flow controller 310, and similarly, the temperature of the cooling medium in the second branch 412 may be controlled by controlling the opening of the ports A, B, and C of the second flow controller 320. In some embodiments, the first flow controller 310 and the second flow controller 320 may be controlled manually or automatically. Exemplarily, when the first flow controller 310 and the second flow controller 320 are automatically controlled, a control module may control a degree of opening of the valve of the first flow controller 310 based on the temperature of the cooling medium flowing to the first electric unit 100 that is collected by the temperature sensor, and control a degree of opening of the valve of the second flow controller 320 based on the temperature of the cooling medium flowing to the second electric unit 200 that is collected by the temperature sensor. In some embodiments, when there are a plurality of branches, a temperature sensor and a flow controller may be disposed in each of the plurality of branches, and the control module may control the degree of opening of the valve of the flow controller corresponding to each temperature sensor based on the temperature of the cooling medium flowing to a corresponding load group that is collected by each temperature sensor.

In some embodiments, to prioritize the cooling medium flowing from the first electric unit 100 to the outlet branch 311, a height (e.g., an absolute height relative to a sea level) of a connecting interface between the outlet branch 311 and the outlet pipeline of the first electric unit 100 may be lower than a height of the outlet end of the first electric unit 100, and the height of the connecting interface between the outlet branch 311 and the outlet pipeline of the first electric unit 100 may be lower than a height of a connecting interface between the outlet pipeline of the first electric unit 100 and the trunk line of the circulation pipeline 40. Similarly, to prioritize the cooling medium flowing out from the second electric unit 200 to the outlet branch 312, a height of a connecting interface between the outlet branch 312 and the outlet pipe of the second electric unit 200 may be lower than the height of the outlet end of the second electric unit 200, and the height of the connecting interface between the outlet branch 312 and the outlet pipeline of the second electric unit 200 may be lower than a height of a connecting interface between the outlet pipeline of the second electric unit 200 and the trunk line of the circulation pipeline 40.

In some embodiments, the first heat exchanger module 430 may include a first heat exchanger (not shown in the figure) and a second heat exchanger (not shown in the figure). The first heat exchanger may be disposed on the first branch 411, and configured to perform temperature adjustment on the cooling medium flowing through the first branch 411, and the second heat exchanger may be disposed on the second branch 412, and configured to perform temperature adjustment on the cooling medium flowing through the second branch 412, so as to more precisely control the temperature of the cooling medium flowing through the first electrical unit 100 and/or the second electrical unit 200. Exemplarily, in some embodiments, the first heat exchanger may be disposed between the first flow controller 310 and the first electric unit 100 in order to enable the first heat exchanger to simultaneously adjust the temperature of the cooling medium flowing from the trunk line of the circulation pipeline 40 and the outlet pipeline of the first branch 411 to the inlet pipeline of the first branch 411. Similarly, the second heat exchanger may be disposed between the second flow controller 320 and the second electric unit 200 in order to enable the second heat exchanger to simultaneously adjust the temperature of the cooling medium that flows from the trunk line of the circulation pipeline 40 and the outlet pipeline of the second branch 412 to the inlet pipeline of the second branch 412.

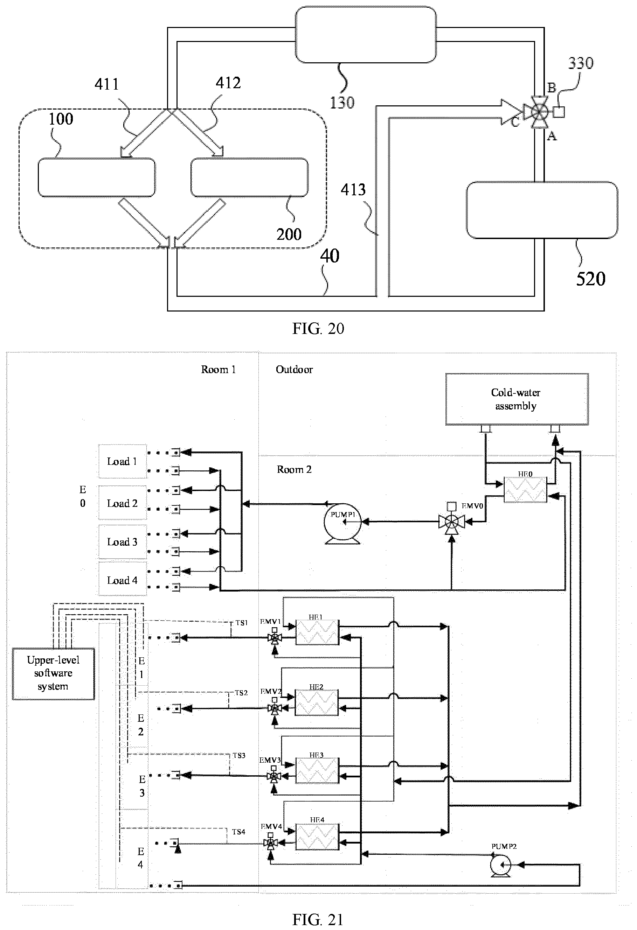

Referring to FIG. 20, in some embodiments, a third flow controller 330 may be disposed on the trunk line of the circulation pipeline 40. Exemplarily, the third flow controller 330 may be connected between the first heat exchange module 430 and the first driving module 130. A port A of the third flow controller 330 may be connected to the first heat exchange module 430, a port B of the third flow controller 330 may be connected to the first driving module 130, and a port C of the third flow controller 330 may be connected to the outlet pipeline of the first branch 411 and/or the second branch 412 by a pipeline 113. By the pipeline 113, the cooling medium flowing out from the outlet line of the first branch circuit 411 and/or the second branch circuit 412 may flow from the third flow controller 330 to the first electric unit 100 and/or the second electric unit 200 again. It may be understood that, in some embodiments, by controlling a degree of opening of each port in the third flow controller 330, a mixing ratio of the cooling medium flowing through the third flow controller 330 may be controlled, so as to control the temperature of the cooling medium flowing to the first electric unit 100 and/or the second electric unit 200.

Referring to FIG. 21, E0 may represent a medical device, and E1, E2, E3, and E4 may represent different medical devices or a plurality of loads (i.e., a to-be-cooled region) in a same medical device. In some embodiments, the E0 to E4 may be disposed in the room 1. HE0, HE1, HE2, HE3, and HE4 may represent heat exchange modules, EMV0, EMV1, EMV2, EMV3, and EMV4 may represent electronic three-way valves, TS1, TS2, TS3, and TS4 may represent temperature sensors, and PUMP1 and PUMP2 may represent driving modules. In the figure, a line between devices may be understood as a cooling pipeline configured to transmit the cooling medium, and an arrow may refer to a flow direction of the cooling medium in the cooling pipeline. In some embodiments, the heat exchange modules HE0 to HE4, the electronic three-way valves EMV0 to EMV4, and the driving modules PUMP1 and PUMP2 may be disposed in the room 2, and a cold-water assembly may be disposed outside. It may be understood that, in this description, the room 1 and the room 2 may be understood as two rooms that are configured to accommodate a medical device and a cooling apparatus related to the medical device.

In some embodiments, the cold-water assembly, the heat exchange module HE0, the driving module PUMP1, the medical device E0, and the electronic three-way valve EMV0 may form a cooling subsystem configured to perform heat dissipation on the medical device E0. In some embodiments, the medical device E0 may include one or more loads. Exemplarily, the medical device E0 may include a load 1, a load 2, a load 3, and a load 4. It may be understood that each load may be disposed in an element or a part of the medical device E0 that requires heat dissipation. It should be noted that, in some embodiments, the load 1, the load 2, the load 3, and the load 4 may be connected in series or parallel by a cooling pipeline, or may be connected in partially series or partially parallel. The electronic three-way valve EMV0 may be connected to an upper-level software system (e.g., a control module). In some embodiments, the upper-level software system may control opening degree of each port of the electronic three-way valve EMV0, so as to adjust the temperature of the cooling medium remaining at the load.

In some embodiments, the heat exchange modules HE1, HE2, HE3, and HE4 may be connected in parallel to the HE0, and the cold-water assembly, the heat exchange HE1, the electronic three-way valve EMV1, the driving module PUMP2, the medical device (or the load) E1, and the temperature sensor TS1 may form a cooling subsystem configured to perform heat dissipation on the medical device (or the load) E1. Similarly, the cold-water assembly, the heat exchange HE2, the electronic three-way valve EMV2, the driving module PUMP2, the medical device (or the load) E2, and the temperature sensor TS2 may form a cooling subsystem configured to perform heat dissipation on the medical device (or the load) E2. The cold-water assembly, the heat exchange HE3, the electronic three-way valve EMV3, the driving module PUMP2, the medical device (or the load) E3, and the temperature sensor TS3 may form a cooling subsystem configured to perform heat dissipation on the medical device (or the load) E3. The cold-water assembly, the heat exchange HE4, the electronic three-way valve EMV4, the driving module PUMP2, the medical device (or the load) E4, and the temperature sensor TS4 may form a cooling subsystem configured to perform heat dissipation on the medical device (or the load) E4. The temperature sensors TS1 to TS4 may be disposed on an inlet pipeline of a load of the medical devices (or the loads) E1 to E4, and configured to detect the temperature of the cooling medium that flows into the load. The upper-level software system may control, based on the temperature detected by the temperature sensors TS1 to TS4, the opening of each valve in the corresponding electronic three-way valve, so as to adjust the temperature of the cooling medium that flows into the load of the corresponding medical device.

In some embodiments, at least one of the electronic three-way valves EMV1 to EMV4 may be connected to an inlet end of a heat exchange module in at least one other cooling subsystem, so that a high-temperature cooling medium obtained after heat exchange with a medical device may flow, by the electronic three-way valve, into a load of a medical device to be cooled in another cooling subsystem. In some embodiments, the upper-level software system may control, based on the temperature detected by the temperature sensor, the opening of each valve in the corresponding electronic three-way valve, so as to control a mixture ratio of the cooling medium flowing out from the heat exchange module to the high-temperature cooling medium flowing in from another cooling subsystem, and further adjust the temperature of the cooling medium flowing in the load of the medical device.

Referring to FIG. 22, in some embodiments, the heat dissipation system may further include a cooling pipeline 440 and a second heat exchange module 432. The cooling pipeline 440 may be configured to transport the cooling medium to a third member 143 disposed in the third medical device 60, such as an electric load. The second heat exchange module 432 may be in communication with the cooling pipeline 440, and configured to adjust the temperature of the cooling medium in the cooling pipeline 440. Similar to the foregoing first heat exchange module 430, in some embodiments, the second heat exchange module 432 may include at least one of a pipe-fin type, a plate-fin type, a microchannel, a titanium pipe type, or the like.

In some embodiments, the third medical device 60 may be a device having a rotation condition, such as a CT device. The third medical device 60 may include a rotating rack. In some embodiments, to meet a heat dissipation requirement of the third medical device 60 in operation, the cooling pipeline 440 and the second heat exchange module 432 may be disposed on the rotating rack of the third medical device 60, so that the cooling pipeline 440 and the second heat exchange module 432 can rotate together with the rotating rack of the third medical device 60.

In some embodiments, the cooling apparatus 100 may further include a second driving module 132, and the second driving module 132 may be connected in the cooling pipeline 440, so as to drive the cooling medium in the cooling pipeline 440. Similar to the first driving module 130, in some embodiments, the second driving module 132 may include various types of driving pumps such as a gear pump, a blade pump, a plunger pump, or the like. In some embodiments, the second driving module 132 may be a shielding pump or a magnetic pump. In some embodiments, the second driving module 132 may be disposed on the rotating rack of the third medical device 60, so that the second driving module 132 can rotate with the rotating rack of the third medical device 60.

In some embodiments, the heat dissipation system may include a connecting pipeline and a winding frame. The connecting pipeline is configured to communicate the cooling pipeline 440 to the circulation pipeline 40, so that the cooling medium in the circulation pipeline 40 flows to the cooling pipeline 440. The connecting pipeline may have a certain flexibility, and may be disposed around the winding frame. In some embodiments, the winding frame may be disposed coaxially with the rotating rack of the third medical device 60, and fixed relative to the rotating rack. When the winding frame rotates with the rotating rack of the third medical device 60, the quantity of circles of the connecting pipeline around the winding frame may be increased or decreased.

In some embodiments, it is considered that the circulation pipeline 40 and the cooling pipeline 440 are connected by a connecting pipeline, not only an infrastructure such as a winding frame needs to be used, which leads to an increase in device cost, but also a large quantity of pipes are needed, so that the connecting pipeline is prone to a fault when rotating with the rotating rack of the third medical device 60, thereby bringing more risks such as leakage and condensation. Based on this, in some embodiments, to avoid the foregoing problem, the cooling pipeline 440 may be disposed as a closed pipeline. Specifically, the cooling pipeline 440 may be an independent circulation loop relative to the foregoing circulation pipeline 40. The cooling pipeline 440 and the circulation pipeline 40 may not be connected and affected with each other in a use process. It should be noted that in the present embodiment of the description, when the cooling pipeline 440 is a closed pipeline, the cooling medium in the cooling pipeline 440 may be the same as or different from the cooling medium in the circulation pipeline 40.

In some embodiments, when the cooling pipeline 440 is a closed pipeline, the cooling pipeline 440 may include an injecting interface for injecting the cooling medium and a discharge interface for discharging the cooling medium. In some embodiments, the injecting interface and the discharge interface may be detachably connected to the circulation pipeline 40, and an exemplary detachable connection manner may include a threaded connection, a jack connection, or the like. When the injecting interface and the discharge interface are connected to a first cooling pipeline, the cooling pipeline 440 and the circulation pipeline 40 may be connected to each other, so that the circulation pipeline 40 provides the cooling medium for the cooling pipeline 440. As an example only, in some embodiments, the injecting interface may be located at an inlet end of the second heat exchange module 432 (e.g., one end of the second heat exchange module 432 where the cooling medium flows in), and the injecting interface may be connected to an outlet end of the first heat exchange module 430 (e.g., one and of the first heat exchange module 430 where the cooling medium flows out). The discharge interface may be located at an outlet end of the second heat exchange module 432 (e.g., one end of the second heat exchange module 432 where the cooling medium flows out), and the discharge interface may be connected to an inlet end of the first heat exchange module 430 (e.g., one and of the first heat exchange module 430 where the cooling medium flows in).

In some embodiments, the injecting interface may be connected to an outlet end of the first electric unit 100 or the second electric unit 200. In this case, heat absorbed by the first electric unit 100 or the second electric unit 200 in a process of performing heat dissipation on the first medical device 210 or the second medical device 220 may preheat the cooling medium flowing into the cooling pipeline 440, so that a difference between the temperature of the cooling medium in the cooling pipeline 440 and an operating temperature of the third medical device 60 is less than a preset threshold (e.g., less than 20 °C), and it is avoided that damage to the third medical device 60 due to a large temperature difference

In some embodiments, the cooling pipeline 440 may be set as the closed pipeline, the winding frame may be avoided to use, and the connecting pipeline does not need to be wound on the winding frame when rotating with the rotating rack, thereby resolving a problem of a large quantity of pipelines and reducing a risk of leakage of the cooling medium.

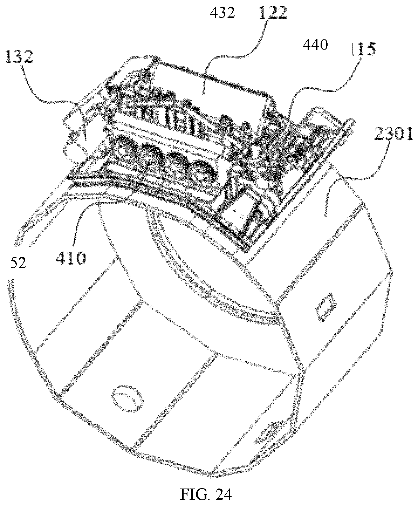

Referring to FIG. 24, in some embodiments, the cooling pipeline 440, the second heat exchange module 432, and the second driving module 132 may be integrated into a subsystem of the cooling apparatus 100, and the subsystem may be disposed on the rotating rack 2301 of the third medical device 60 (such as a CT device) with a rotating state, so as to perform heat dissipation on the third medical device 60.

In some embodiments, it is considered that a leakage problem may exist in a case that a conventional mechanical shaft seal pump rotates at a high speed with the rotating rack 2301, to avoid a leakage problem of the second driving module 132, in some embodiments, the second driving module 132 may apply a shielded pump without a shaft seal. Similarly, in some embodiments, an aluminum microchannel plate-fin heat exchanger may be used to avoid a deformation failure of a copper pipe and a fin caused by a centrifugal force of the second heat exchange module 432 under high-speed rotation. In some embodiments, a control circuit of the heat dissipation system may include a relay. Similarly, to avoid a centrifugal force failure of the relay under high-speed rotation, a solid-state relay may be applied in the control circuit of the heat dissipation system.

In some embodiments, the heat dissipation system may further include a first fan 52, and the first fan 52 may be fastened to the rotating rack 2301, so as to change an airflow speed on a surface of the second heat exchange module 432, so as to perform heat dissipation on the second heat exchange module 432 in a form of air cooling. In some embodiments, the first fan 52 may include air cooling devices such as a fan and an air conditioner. In some embodiments, a rotating axis of the first fan 52 may be parallel to a rotating axis of the third medical device 60, thereby reducing an effect of a centrifugal force on a motor in the first fan 52 in a rotation process.

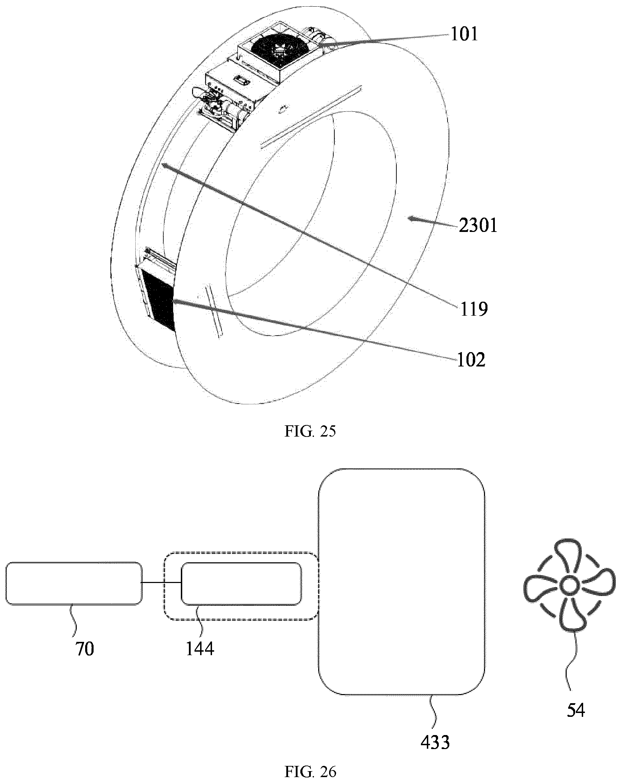

Referring to FIG. 25, in some embodiments, the heat dissipation system may include a heat exchange module 101 and a water-cooling plate 102. The heat exchange module 101 may be connected to the water-cooling plate 102 by using a connecting pipeline 119 to form a circuit. In some embodiments, the heat exchange module 101 and the water-cooling plate 102 may be disposed on the rotating rack 2301 of a medical device (e.g., the third medical device 60) with a rotating condition. The heat exchange module 101 may output a low-temperature cooling medium, and transport the low-temperature cooling medium to the water-cooling plate 102 by the connecting pipeline 119. The cooling medium in the water-cooling plate 102 may be heat exchanged with a heat consumption member (e.g., the third medical device 60), so as to perform heat dissipation on the heat consumption member. The cooling medium output by the water-cooling plate 102 may become a high-temperature cooling medium after heat exchange with the heat consumption member. Furthermore, the high-temperature cooling medium may flow back to the heat exchange module 101, cool down after heat exchange with the heat exchange module 101 to obtain a low-temperature cooling medium, and then be transported to the water-cooling plate 102 by the connecting pipeline 119 again. In this way, heat dissipation may be performed on the heat dissipation member that is in contact with the water-cooling plate 102.

The heat exchange module 101 may include at least one of various heat exchangers such as a pipe-fin type, a plate-fin type, a microchannel, or a titanium pipe type. In some embodiments, the heat exchange module 101 may include a heat dissipation fan, and the heat dissipation fan is configured to change an air flow speed on a surface of the heat exchange module 101, so as to cool, by means of air cooling, the high-temperature cooling medium flowing into the heat exchange module 101.

Referring to FIG. 26, in some embodiments, the heat dissipation system may further include a third heat exchange module 433 and a second fan 54. The third heat exchange module 433 may be in thermal conduction with a fourth member 144 of the fourth medical device 70, such as an electrical load, and the second fan 54 is configured to change an airflow speed on a surface of the third heat exchange module 433, so as to perform heat dissipation on the fourth medical device 70 in an air-cooling manner.

The thermal conduction between the third heat exchange module 433 and the fourth component 144 may be understood as a heat transfer relationship between the third heat exchange module 433 and the fourth component 144. For example, in some embodiments, the third heat exchange module 433 and the fourth component 144 may be in direct or indirect contact.

In some embodiments, the fourth medical device 70 may be a device that generates relatively small heat in operation (e.g., an operating temperature is less than 50 °C), such as a US device or a CT device. The third heat exchange module 433 may be an element with good thermal conductivity, and may contact a part that needs to be cooled in the fourth medical device 70. In some embodiments, the third heat exchange module 433 may be made of a material with a relatively high thermal conductivity such as silver, copper, aluminum, alloy, graphene, and carbon fiber composite material. In some embodiments, the third heat exchange module 433 may have a large surface area (e.g., may be more than twice a contact area between the third heat exchange module 433 and the fourth component 144), so as to increase a contact area with the air and ensure good heat dissipation efficiency.

In some embodiments of this description, to increase heat dissipation efficiency of the third heat exchange module 433, the second fan 54 may be used to change the airflow speed on the surface of the third heat exchange module 433. In some embodiments, the second fan 54 may include an air-cooling device such as a fan, an air conditioner, or the like.

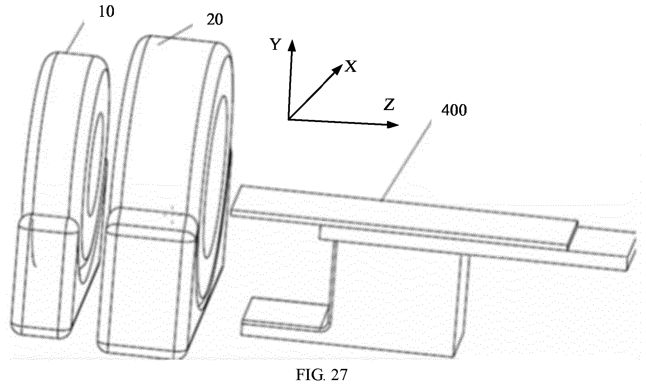

Referring to FIG. 27, in some embodiments, a rack of the medical device may include a housing of the medical device that is configured to accommodate the electric unit, and the housing may form a support structure that is configured to carry the load member. In some embodiments, a shape of the housing may be, for example, an annular shape, and the medical system further includes a transmission apparatus 400 configured to transfer a patient to an imaging region in a center of an annular housing.

The heat dissipation system in the present embodiment of the present invention may include the cooling device 30 and the circulation pipeline 40 with the foregoing structure, so that when the heat dissipation system is applied to a medical system and is in operation, simultaneous or alternating heat dissipation may be performed on the medical device in a heat exchange manner of the cooling medium, thereby improving heat dissipation efficiency of the heat dissipation system, reducing heat dissipation costs, reducing noise when the medical system that applies the heat dissipation system is in operation, improving user experience, and reducing a probability that foreign objects such as dust enters inside the heat dissipation system, so that the heat dissipation system may be applicable to a high-end medical system.

A medical system is further provided in an embodiment of the present invention, including the heat dissipation system and the at least two electric units.

In some embodiments, the at least two electric units may include at least two different medical devices, thereby forming a multi-mode medical system. In some embodiments, the at least two medical devices may be selected from at least one of a CT device, an MR device, a PET device, an RT device, an Ultrasound diagnostics device, a Single-Photon Emission Computed Tomography device, or an X-ray imaging device, respectively.

In a specific embodiment, the medical system may include a plurality of different types of medical devices and a heat dissipation system that can perform different heat dissipation for different medical devices. For example, the medical system may include all of an MR device, a PET device, a RT device, a CT device, an ultrasound diagnostic device, and other apparatus devices. The heat dissipation system may include a circulation pipeline 40, a cooling device 30, a first fan 52, and a second fan 54. The first fan 52 may be, for example, a fan. The second fan 54 may be, for example, an indoor air conditioner. For the MR device and the PET device, the heat dissipation system may transmit liquid medium by a closed circulation pipeline 40, and perform liquid-cooling heat dissipation on the MR device and the PET device. For the RT device and the CT device, the heat dissipation system may perform air-cooling heat dissipation on the RT device and the CT device by a fan disposed on the rotating rack 2301. For the ultrasound diagnostics device and other apparatus devices in the medical system, the heat dissipation system may lower an indoor temperature by the indoor air conditioner, so as to perform heat dissipation on these devices. Various medical devices, the first fan 52, and the second fan 54 may be disposed in a same room, a heat exchanger of the cooling device 30 may be disposed in another room inside, and the circulation pipeline 40 may be disposed in the room, and connected to the cooling device 30 and the medical device in the two rooms. The cooling device 3 and the air conditioner may share a condenser which is disposed outside. The medical system may implement multi-path heat dissipation for different types of medical devices by the heat dissipation system. Air-cooling heat dissipation may be applied for the RT device and the CT device with a rotating state, so as to reduce mounting difficulty of the circulation pipeline 40. For an ultrasound diagnostics device and other apparatus devices that have relatively small heat generation, air-conditioning heat dissipation may be applied, and costs may be reduced. An actual heat dissipation requirement of each medical device may be integrated, and the simplest and most effective heat dissipation solution may be provided, so as to solve a problem of multi-operation and multi-load heat dissipation.

All the technical features in the foregoing embodiments may be combined randomly. To make the description brief, all possible combinations of the technical features in the foregoing embodiments are not described. However, as long as there is no contradiction between the combinations of the technical features, it should be considered as the scope described in this description.

The foregoing embodiments represent only several implementation manners of the present invention, and descriptions thereof are relatively specific and detailed, but may not be construed as a limitation on the scope of patent protection. It should be noted that one skilled in the art may make some modifications and improvements without departing from the concept of the present invention, which are within the protection scope of the present invention. Therefore, the protection scope of the present invention shall be subject to the attached claims.

## Claims

1. A heat dissipation system configured for a medical system, **characterized by** comprising a circulation pipeline (40) and a cooling device (30),
wherein the circulation pipeline (40) is configured for conveying a cooling medium to the medical system, the medical system includes at least two electric units, and the at least two electric units have different rated cooling temperatures; and
the cooling device (40) is in communication with the circulation pipeline (40) and is configured for adjusting a temperature of the cooling medium, so as to dissipate heat of the at least two electric units.

2. The heat dissipation system of claim 1, wherein the at least two electric units comprise at least two medical devices, which comprise a first medical device (10) and a second medical device (20).

3. The heat dissipation system of claim 2, wherein a rated cooling temperature of the first medical device (10) is lower than that of the second medical device (20), the second medical device (20) is connected to the first medical device (10) in series by the circulation pipeline (40), so that at least a part of the cooling medium flowing out from the first medical device (10) flows into the second medical device (20).

4. The heat dissipation system of claim 3, wherein the first medical device (10) is in communication with the second medical device (20) and the cooling device (30) by the circulation pipeline (40), respectively, so that a part of the cooling medium flowing out from the first medical device (10) flows into the second medical device (20), and the other of the cooling medium directly flows back to the cooling device (30).

5. The heat dissipation system of claim 4, wherein a flow adjusting apparatus (50) is disposed in the circulation pipeline (40), and is configured to adjust a flow ratio of a part of the cooling medium which flows into the second medical device (20) to a part of the cooling medium which directly flows back to the cooling device (30).

6. The heat dissipation system of any one of claims 3 to 5, wherein the first medical device (10) is provided with a first flow path (11), the second medical device (20) is provided with a second flow path (21), and the first flow path (11) and the second flow path (21) are in communication with the cooling device (30) in series by the circulation pipeline (40).

7. The heat dissipation system of claim 6, wherein the circulation pipe (40) comprises a first liquid outlet pipe (41), a first branch pipe (42), and a first return pipe (43), a liquid inlet end of the first flow path (11) is in communication with the first liquid outlet pipe (41), a liquid outlet end of the first flow path (11) is in communication with a liquid inlet end of the second flow path (21) by the first branch pipe (42), and a liquid outlet end of the second flow path (21) is in communication with the first return pipe (43).

8. The heat dissipation system of claim 7, wherein the circulation pipeline (40) further comprises a second branch pipe (44), and the second branch pipe (44) and the second flow path (21) are connected in parallel between the first flow path (11) and the cooling device (30), or are connected in parallel between the first flow path (11) and the first return pipe (43).

9. The heat dissipation system of claim 2, wherein the second medical device (20) is connected to the first medical device (10) in parallel by the circulation pipeline (40), so that the cooling medium flows into the first medical device (10) and the second medical device (20), respectively.

10. The heat dissipation system of claim 9, wherein the first medical device (10) is provided with a first flow path (11), the second medical device (20) is provided with a second flow path (21), and the first flow path (11) and the second flowpath (21) are in communication with the cooling device (30) in parallel by the circulation pipeline (40).

11. The heat dissipation system of claim 10, wherein the circulation pipeline (40) comprises a second liquid outlet pipe (410) and a second return pipe (420), a liquid inlet end of the first flow path (11) and a liquid inlet end of the second flow path (21) are in communication with the second liquid outlet pipe (410) in parallel, and a liquid outlet end of the first flow path (11) and a liquid outlet end of the second flow path (21) are in communication with the second return pipe (420) in parallel.

12. The heat dissipation system of claim 10, wherein the circulation pipeline (40) comprises a third liquid outlet pipe (401), a fourth liquid outlet pipe (402), a third return pipe (403), and a fourth return pipe (404), the first flow path (11) is in communication with the cooling device (30) by the third liquid outlet pipe (401) and the third return pipe (403), and the second flow path (21) is in communication with the cooling device (30) by the fourth liquid outlet pipe (402) and the fourth return pipe (404).

13. The heat dissipation system of any one of claims 9 to 12, wherein the at least two medical devices comprise a third medical device (60), and both a rated cooling temperature of the first medical device (12) and a rated cooling temperature of the second medical device (22) are greater than that of the third medical device (60); and
the third medical device (60) is connected to the first medical device (10) and the second medical device (20) in series by the circulation pipeline, the third medical device (60) is disposed in front of the first medical device (10) and the second medical device (20), so that at least a part of the cooling medium flows out from the third medical device (60) flows into the first medical device (10) and the second medical device (20).

14. The heat dissipation system of any one of claims 9 to 13, wherein the at least two medical devices comprise a fourth medical device (70), and both a rated cooling temperature of the first medical device (12) and a rated cooling temperature of the second medical device (22) are less than that of the fourth medical device (70); and
the fourth medical device (70) is connected to the first medical device (10) and the second medical device (20) in series by the circulation pipeline, the fourth medical device (70) is disposed beyond the first medical device (10) and the second medical device (20), so that at least a part of the cooling medium flows out from the first medical device (10) and the second medical device (20) flows into the fourth medical device (70).

15. The heat dissipation system of any one of claims 6 to 8 and claims 10 to 12, wherein the first flow path (11) comprises a plurality of first branch flow paths (111) which are disposed in parallel in the first medical device (10).

16. The heat dissipation system of any one of claims 6 to 8 and claims 10 to 12, wherein the second flow path (21) comprises a plurality of second branch flow paths (211) which are disposed in parallel in the second medical device (20).

17. The heat dissipation system of any one of claims 1 to 16, wherein in the at least two medical devices, a medical device with a low rated cooling temperature is disposed in front of a medical device with a high rated cooling temperature, so that the cooling medium always flows from the medical device with the low rated cooling temperature into the medical device with the high rated cooling temperature.

18. The heat dissipation system of claim 1, wherein the at least two electric units comprise at least two load members of a same medical device, and the at least two load members comprise a first member (141) and a second member (142).

19. The heat dissipation system of claim 18, wherein the circulation pipeline (40) is connected to the first member (141) and the second member (142) in series, and the circulation pipeline (40) is capable of conveying the cooling medium to the first member (141) and the second member (142), respectively, wherein a rated cooling temperature of the first member (141) is less than a rated cooling temperature of the second member (142).

20. A medical system, **characterized by** comprising the heat dissipation system of any one of claims 1 to 19 and the at least two electric units.

21. The medical system of claim 20, wherein the at least two electric units comprise at least two medical devices, which comprise at least one of a Computed Tomography device, a Magnetic Resonance imaging device, a Positron Emission Tomography device, a radiotherapy device, an Ultrasound diagnostics device, a Single-Photon Emission Computed Tomography device, or an X-ray imaging device, respectively.
